# EUROPÄISCHE PATENTSCHRIFT

(11) **EP 3 203 896 B1**
(45) Veröffentlichungstag und Bekanntmachung des Hinweises auf die Patenterteilung: **13.03.2019**
(21) Anmeldenummer: 16748130.8
(22) Anmeldetag: 09.08.2016
(51) Int. Cl.: A61B 1/00, A61B 1/005, G02B 23/24, A61B 17/00

(54) **ENDOSKOP MIT LÖSBAREM HANDGRIFF**
ENDOSCOPE WITH SEPARABLE HANDLE
ENDOSCOPE AVEC POIGNÉE SÉPARABLE

(30) Priorität: 14.08.2015 DE 102015113424
(43) Veröffentlichungstag der Anmeldung: 16.08.2017
(73) Patentinhaber: Ambu A/S, 2750 Ballerup (DK)
(72) Erfinder: CIFARELLI, John F., Oyster Bay, New York 11771 (US); HERCEGFI, Timo, 69221 Dossenheim (DE); FIEBER, Markus, Dr., 80636 München (DE); ABICHT, Felix, 82337 Walchstadt (DE); SCHÜTZ, Günter, 86152 Augsburg (DE); ILG, Dylan, 82266 Inning (DE); POMMERSHEIM, Wilhelm, 86447 Adelsried (DE); AFHOLDERBACH, Tim, 86165 Augsburg (DE)
(74) Vertreter: Winter, Brandl, Fürniss, Hübner, Röss, Kaiser, Polte - Partnerschaft mbB
(86) Internationale Anmeldenummer: PCT/EP2016/068951
(87) Internationale Veröffentlichungsnummer: WO 2017/029156

(56) Entgegenhaltungen:
- JP-A- H0 815 614
- US-A1- 2002 103 418
- US-A1- 2007 060 789
- US-A1- 2008 064 925
- US-A1- 2008 200 758
- US-A1- 2011 251 459
- US-A1- 2014 107 416
- US-A1- 2014 275 763
- US-A1- 2014 288 460

## Beschreibung

Die vorliegende Offenbarung betrifft den als separates, adaptives Bauteil ausgebildeten Handgriff eines Endoskops (einschließlich Koloskop und/oder Gastroskop) mit Optik, Beleuchtung sowie einem Arbeitskanal mit Endoskop-spezifischer Funktion und Dimensionierung gemäß dem Oberbegriff des Patentanspruchs 1.

### Hintergrund der Offenbarung

Endoskope sind medizinische Arbeitsgerätschaften, mittels denen das Innere von in der Regel lebenden Organismen, aber auch technischen Hohlräumen untersucht und/oder manipuliert werden kann. Ursprünglich für die humanmedizinische Diagnostik entwickelt, werden sie heute auch für minimal-invasive operative Eingriffe an Mensch und Tier sowie in der Industrie zur Sichtprüfung schwer zugänglicher Hohlräume eingesetzt. Zu den Endoskopen zählen die starren und flexiblen Endoskope und deren Unterarten.

Ein starres Endoskop leitet die Bildinformationen des zu untersuchenden Objekts bzw. Raums durch ein Linsensystem im Inneren des Endoskopschafts bzw. im distalen Endoskopkopf an ein proximal angeordnetes Okular weiter. Beispiele hierfür sind das technische Boreskop und med. das Arthroskop und Zystoskop.

Das für die Untersuchung/Inspektion notwendige Licht wird entweder durch ein distales Leuchtmittel (z.B. LED) im Endoskopkopf bereitgestellt oder über einen an ein proximales Leuchtmittel angeschlossenen Lichtleiter, ebenfalls im Inneren des Schafts durch Glasfaserbündel an die Spitze des Endoskops transportiert.

Bei einem flexiblen Endoskop werden Bild und Licht ebenfalls häufig über Glasfaserbündel übertragen. Beispiele sind das technische Flexoskop und das med. Gastroskop, Koloskop und Bronchoskop.

Ab einem praktikablen Durchmesser sind flexible (und auch starre) Endoskope auch mit auswechselbaren statt festmontierten Objektiven (*Vor*/*Seit* oder *Rückwärts*) sowie zusätzlichen Arbeitskanälen zum Einführen von mikromechanischen Geräten (kleine Zangen oder Greifer) in den Untersuchungs- bzw. Inspektionsraum erhältlich. Flexible Endoskope besitzen meist eine über eingebaute Bowdenzüge oder Hydraulik-Stellantriebe fernsteuerbare Gerätespitze (Endoskopkopf). Diese kann je nach Modell und Durchmesser nach 2 (*auf-ab*) oder nach 4 Seiten (*auf-ab* und *rechts-links*) teilweise bis zu 180° abgewinkelt werden. Die Länge dieser abwinkelbaren Spitze kann je nach Durchmesser zwischen 30 und 70 mm liegen. Im proximalen Handgriff des Geräts/Endoskops ist eine Mechanik eingebaut, die über Kippbügel oder Handräder auf die Bowdenzüge/Stellantriebe einwirkt und die Bewegung der Spitze ermöglicht.

Die jüngste Unterart der flexiblen Endoskope bilden die Videoendoskope, oft auch Videoskope genannt. Videoendoskope eröffnen ein neues Kapitel in der modernen Endoskopie, da sie zur Bilderzeugung und -übertragung digitale Technologien nutzen. Ein am distalen Objektiv des Videoendoskops angebrachter CCD- bzw. CMOS-Chip erzeugt ein digitales Bild des Untersuchungsobjekts und leitet es an die folgenden Baugruppen des Videoendoskops weiter. Meist bereitet dann ein Prozessor diese Daten auf, sendet sie zur Ausgabe an einen Monitor, oder legt sie auf einer Festplatte oder anderen Datenspeichern ab. Videoendoskope ermöglichen je nach Ausstattungsvariante das Einfrieren und Speichern von Bildern sowie mehrstündige Videoaufzeichnungen für eine nachträgliche Aufbereitung/Optimierung oder auch das Vermessen eines Bildes bzw. Objektes. Auch diese Gerätetechnik besitzt eine fernsteuerbar, abwinkelbare Gerätespitze, nach 2 oder 4 Richtungen. Diese können mechanisch oder elektronisch/hydraulisch gesteuert werden. Die mechanische Steuerung erfolgt über ein kleines Getriebe über Kipphebel oder Drehräder. Einige Videoskope haben anstelle der Mechanik kleine Elektromotoren oder Hydraulikbalge eingebaut, die beispielsweise über einen Joystick ansteuerbar sind.

Neuerdings kommen bei Videoskopen anstelle der externen Lichtprojektoren, auch LED-Leuchtkörper zum Einsatz. Diese können in der Gerätespitze eingebaut sein, oder auch im proximalen Handgriff. Im letzteren Fall wird dann das Licht über eingebaute Glasfasern nach vorn zur Spitze geleitet.

Zusammenfassend sind demnach starre wie auch flexible Endoskope der einschlägigen Gattung (einschließlich des vorliegenden Offenbarungsgegenstands) gekennzeichnet durch einen Endoskopschaft (flexibel oder starr), an dessen distalem Ende (Endoskopkopf) eine Beleuchtung (Lichtaustritt eines Lichtleiterkabels oder ein Leuchtkörper) sowie eine Optik (Linsensystem und/oder lichtempfindlicher Chip) angeordnet sind. Innerhalb des Endoskopschafts ist (neben optionalen diversen Funktions-/Versorgungskanälen beispielsweise für die Optik, die Beleuchtung und/oder die Abkrümmeinrichtung der Instrumentenspitze) ein Arbeitskanal ausgeformt, der dafür vorgesehen und angepasst ist, chirurgische Instrumente durch das Endoskop hindurch in den Patientenkörper einzuführen, um unter visueller Kontrolle über die Optik und die Beleuchtung (minimalinvasive) Operationen/Behandlungen vorzunehmen.

Da chirurgische Instrumente für minimalinvasive Eingriffe eine Mindestgröße aufweisen, hat der Arbeitskanal einen entsprechenden Durchmesser, der das Einführen der chirurgischen Instrumente erlaubt. Dieser Durchmesser ist in der Regel deutlich größer als der der Funktions-/Versorgungskanäle.

Neben der vorstehend beschriebenen Funktion hat der Arbeitskanal aber auch die Aufgabe, beispielsweise Behandlungsflüssigkeiten (Medikamentlösungen, etc.) zu fördern oder Patientengewebe und/oder Körperflüssigkeiten abzutransportieren. Außerdem kann über den Arbeitskanal Spülflüssigkeit an die Behandlungsstelle im Patientenkörper gepumpt werden.

### Stand der Technik

Aus dem Stand der Technik sind allgemein Endoskope der vorstehenden Gattung (starre und flexible Endoskope) bekannt, bei denen der Arbeitskanal sowohl an der distalen Endoskopspitze wie auch im Bereich des proximalen Handgriffs jeweils eine Austrittsöffnung hat. Über die proximale Öffnung kann dann beispielsweise ein chirurgisches Instrument eingeführt werden oder es kann eine Druck-/Saugpumpe angeschlossen werden.

Das Problem dieser Endoskope besteht jedoch darin, dass insbesondere bei minimalinvasiven chirurgischen Eingriffen bei in den Arbeitskanal eingeführtem chirurgischem Instrument gleichzeitig beispielsweise Spüllösung an die Operationsstelle herangeführt werden muss. Dies erfolgt dann über einen zum Arbeitskanal separat im Endoskopschaft ausgebildeten Versorgungskanal mit vergleichsweise kleinem Durchmesser. Es hat sich hier aber gezeigt, dass nur wenig Spüllösung über diesen engen Versorgungskanal pro Zeiteinheit gefördert werden kann, was sich insbesondere bei besonders langen Endoskopschäften (bis zu 250cm Länge) insoweit negativ auswirkt, als dass die Spüllösung mit hohem Druck in den Versorgungskanal gepumpt werden muss, um dieses in ausreichender Menge pro Zeiteinheit bis zur distalen Endoskopspitze fördern zu können.

Aus den Dokumenten US 2007/060789 A1, US 2014/107416 A1 und US 2014/275763 A1 ist jeweils ein Endoskop bekannt, welches mittels einer kabellosen Technologie mit einer das Endoskop steuernden Bedienstation in Verbindung stehen.

Die Dokumente US 2002/103418 A1 und US 2011/251459 A1 offenbaren jeweils ein Endoskop mit einem Handgriff, welcher eine Aufnahme für einen Endoskopschaft ausbildet.

Weiterer Stand der Technik ist aus den Dokumenten US 2008/200758 A1, JP H08 15614 A, US 2008/064925 A1 und US 2014/288460 A1 bekannt.

### Kurzbeschreibung der Offenbarung

Angesichts dieser, von der vorliegenden Anmelderin erkannten Problematik ist es die Aufgabe der Offenbarung, ein Endoskop dieser Gattung (vorzugsweise flexibel aber ggf. auch starr) bereitzustellen, mittels dem eine ausreichende Flüssigkeitsver- und/oder -entsorgung auch bei gleichzeitig in den Arbeitskanal eingeführtem chirurgischen Instrument möglich ist.

Ferner soll vorzugsweise die Handhabung des Endoskops verbessert werden, indem auf einfache Weise Teile des Endoskops als Ein-Weg-Artikel nach Gebrauch einfach entsorgt und andere Teile des Endoskops als Mehr-Weg-Artikel nach Gebrauch auf einfache Weise gereinigt werden können. Schließlich soll weiter vorzugsweise der Betrieb des Endoskops verbessert werden.

Diese Aufgabe wird erfindungsgemäß durch ein Endoskop mit den Merkmalen des Anspruchs 1 gelöst. Vorteilhafte Ausgestaltungen der Erfindung sind Gegenstand der Unteransprüche. Ein Kerngedanke der Offenbarung ist es demnach, den Arbeitskanal zum Zwecke des Einführens von chirurgischen Instrumenten aber auch des Zu- und/oder Abführens von Fluiden/Flüssigkeiten besser zu nutzen. Die gegenüber dem Stand der Technik verbesserte Ausnutzung des Arbeitskanals wird dadurch erreicht, indem dem Arbeitskanal mindestens zwei proximale Anschlüsse zugeordnet sind, welche separat und unabhängig voneinander (damit auch parallel/gleichzeitig) genutzt werden können, um jeweils ein Medium (ein chirurgisches Instrument und/oder ein Fluid) über jeden proximalen Anschluss in den Arbeitskanal (parallel/gleichzeitig) ein- und/oder abzuführen.

In anderen Worten ausgedrückt hat der (einzige) Arbeitskanal des Endoskops wenigstens zwei proximale Anschlüsse, von denen wenigstens ein proximaler Anschluss für das Einführen eines (medizinischen) Gegenstands wie chirurgisches Instrument geeignet und vorzugsweise angepasst ist und wenigstens ein anderer proximaler Anschluss für das Einführen/Entnehmen eines Fluids wie eine Flüssigkeit (z.B. Spüllösung, Körperflüssigkeit, etc.) und/oder ein Gas (z.B. Luft zum Entfalten eines Patientendarms) geeignet und vorzugsweise angepasst ist.

Auf diese Weise kann der Arbeitskanal auch bei eingeschobenem/eingeführtem chirurgischen Instrument gleichzeitig zur Zufuhr und/oder Entnahme von Fluid genutzt werden. Da folglich ein im Stand der Technik hierfür gesondert vorgesehener Ver- / Entsorgungskanal entfallen kann, lässt sich der Arbeitskanal vergleichsweise großzügig dimensionieren, ohne dass der Endoskopschaft-Außendurchmesser hierfür aufgeweitet werden müsste.

Die Anpassung des einen Anschlusses für das Einführen eines chirurgischen Instruments definiert sich im einfachsten Fall im Anschluss-Inndurchmesser, der so groß sein muss, dass ein chirurgisches Instrument hineingeschoben werden kann. In anderen Worten ausgedrückt orientiert sich der Anschluss-Durchmesser am Durchmesser des Arbeitskanals. Außerdem sollte dieser Anschluss vorzugsweise einen sanften Eingang zum Arbeitskanal aufweisen, also etwa einen inneren Bogen ausführen oder axial zum Arbeitskanal verlaufen, damit das einzuführende chirurgische Instrument nicht abgeknickt wird.

Die Anpassung des Anschlusses für das Ein-/Ausführen eines Fluids definiert sich hingegen im einfachsten Fall durch die Ausbildung einer Verschlussmöglichkeit. In anderen Worten ausgedrückt hat dieser Anschluss eine zusätzliche Einrichtung, an welcher beispielsweise ein Schlauch (fluiddicht) angeschlossen werden kann. Z.B. kann diese ein Rohrstutzen/Nippel sein, auf welchem ein Schlauch aufgesteckt werden kann, oder ein Bajonett-, Schraub- oder Lueranschluss.

Vorzugsweise werden die mehreren Anschlüsse des Arbeitskanals durch einen weiter vorzugsweise einstückigen Anschlusseinsatz gebildet. Dieser Anschlusseinsatz hat (besteht) in einem bevorzugten Ausführungsbeispiel einen Rohrlauf, dessen eines Ende mit dem Arbeitskanal (fluid-)verbunden (verbindbar) ist und an dessen anderem Ende ein Anschlussstutzen angeordnet ist mit einer Anzahl von Anschlussrohren, die sämtlich in den Rohrlauf einmünden. Vorzugsweise ist der Anschlusseinsatz fest mit dem Endoskopschaft an dessen proximalem Ende verbunden. Sofern der Endoskopschaft biegeflexibel ist, weist der Anschlusseinsatz deutlich starrere (steifere) Eigenschaften auf.

Vorteilhafter Weise ist der Rohrlauf dafür angepasst, in einen Handgriff eingesetzt zu werden, derart, dass die Anschlussrohre des Anschlussstutzens zumindest abschnittsweise aus dem Handgriff herausragen, bzw. von außen zugänglich sind. Der Handgriff ist somit von den Anschlüssen separiert und wird daher nicht oder nur geringfügig kontaminiert.

Gemäß einem ggf. unabhängig zu beanspruchenden Aspekt der vorliegenden Offenbarung ist der Handgriff als (lösbarer) Aufsteckhandgriff ausgebildet, der mit dem Anschlusseinsatz und mit dem Rohrlauf des Anschlusseinsatzes vorzugsweise klemmend zusammenwirkt. D.h. das erfindungsgemäße Endoskop besteht im Wesentlichen aus zwei Baueinheiten, nämlich dem Endoskopschaft (mit dem vorzugsweise flexiblen, in den Patientenkörper einzuführenden Schaftabschnitt mit Endoskopkopf und den darin verbauten Funktionen sowie mit dem vorzugsweise starren Anschlusseinsatz) und dem separat hierzu ausgebildeten Handgriff, der einfach am Endoskopschaft (Anschlusseinsatz) temporär fixiert werden kann.

Hierfür hat der Rohrlauf des Anschlusseinsatzes eine Anzahl von Rastelementen oder Hinterschneidungen, die mit entsprechenden Rastelementen oder Hinterschneidungen auf Seiten des Handgriffs zusammenwirken. Vorzugsweise bildet der Handgriff einen Clip-/Schalenabschnitt mit einer Anzahl von Hinterschneidungen und/oder Vorsprüngen, derart, dass der Handgriff auf den Rohrlauf aufgeclipt/aufgeklippt werden kann, wobei entsprechende Vorsprünge und/oder Hinterschneidungen auf Seiten des Clipabschnitts mit den Hinterschneidungen und/oder Vorsprüngen am Rohrlauf in Formschluss kommen. Dadurch wird ein sicherer (aber lösbarer) Halt des Handgriffs auf dem Rohrlauf gewährleistet.

Wie vorstehend bereits angedeutet wurde, ist der Anschlusseinsatz fest mit dem für das Einführen in den Patientenkörper vorgesehenen (biegeflexiblen) Endoskopschaftabschnitt gekoppelt. Der Endoskopschaft (einschließlich des Anschlusseinsatzes) ist hierbei vorzugsweise als Einweg-Gegenstand konzipiert. Da aber der Handgriff keinen unmittelbaren Kontakt beispielsweise mit Körperflüssigkeit des Patienten bekommt (sämtlich Leitungen und Anschlüsse sind im Endoskopschaft), kann dieser als Mehrweg-Produkt vorgesehen sein. Daher ist die Kopplung zwischen Handgriff und Anschlusseinsatz lösbar, vorliegend vorzugsweise als Clickverbindung gedacht. Es könnte aber jedwede andere lösbare Kopplung wie Einsteck-, Klett, Spann- oder Schraubverbindung vorgesehen sein.
am Anschlusseinsatz sind weitere Anschlüsse für die Versorgungs-/Funktionskanäle und/oder die Funktionen wie Optik, Beleuchtung, Stellantriebe, etc. ausgebildet. Auf diese Weise, werden alle Funktionen des Endoskopschafts einschließlich der im Endoskopkopf verbauten Funktion wie Optik, Beleuchtung, Stellantriebe, etc. unmittelbar am Anschlusseinsatz unter Umgehung des Handgriffs angeschlossen.

Gemäß einem anderen, unabhängig beanspruchbaren Aspekt der vorliegenden Offenbarung hat der Handgriff eine Betätigungseinrichtung, beispielsweise ein Tastenfeld, zur Aktuierung einer (separaten) Betriebsstation, welche an das Endoskop über die vorstehend genannten weiteren Anschlüsse angeschlossen/anschließbar ist. Die Steuerungs-Verbindung zwischen Handgriff und Betriebsstation erfolgt vorzugsweise kabellos insbesondere per W-Lan, Blue-Tooth oder Infrarot-Signal, wobei natürlich aber auch eine Kabelverbindung vorgesehen sein kann.

Weiter vorzugsweise ist am Handgriff ein Sensor oder dergleichen Erfassungsschalter angebracht, der den Anschlusseinsatz erfasst, wenn dieser mit dem Handgriff gekoppelt ist, um daraufhin die Aktuierung der Betriebsstation freizuschalten. Zusätzlich oder alternativ kann an den weiteren Anschlüssen ein ähnlicher Sensor/Erfassungsschalter mit gleicher Funktion und Wirkung angeordnet sein.

Gemäß einem anderen, ggf. unabhängig beanspruchbaren Aspekt der vorliegenden Offenbarung bildet das Gehäuse/Aufnahmegehäuse des Handgriffs eine fluiddichte Ummantelung sowohl des Tastenfelds wie auch der darin aufgenommenen Elektronik. Im Fall einer Kabelverbindung ist auch der Anschluss zwischen Kabel und Handgriff vorzugsweise fluiddicht ummantelt. Der Handgriff bildet somit eine fluiddichte Einheit, die separat zum Endoskopschaft auf einfache Weise, beispielsweise durch ein Tauchverfahren, gereinigt werden kann.

An dieser Stelle sei auf die vorteilhafte Ausbildung der Offenbarung hingewiesen, wonach im Wesentlichen alle Funktionen des Endoskops durch die Betriebsstation aktuiert werden. Hierzu zählen weiter vorzugsweise das Aktuieren der Optik und deren Beleuchtung, das Aktuieren des distalen Abwinklungsabschnitts zur kontrollierten Abkrümmung/Abwinklung des Endoskops in dessen distalem Längenabschnitt, das Aktuieren des Einpressens von Spüllösungen oder Medikamenten oder weiteren Fluiden und das Aktuieren des Absaugens von Flüssigkeiten/Fluiden. Diese Funktionen sind mittels des Bedienfelds vorzugsweise elektrisch steuerbar, wofür das Bedienfeld über eine Signalleitung (beispielsweise W-Lan, Blue-Tooth, Infrarot oder Kabel) mit der Betriebsstation gekoppelt ist, welche mit der Kommunikationseinrichtung verbunden ist.

### Figurenbeschreibung

Die vorliegende Erfindung wird nachfolgend anhand eines bevorzugten Ausführungsbeispiels unter Bezugnahme auf die begleitenden Figuren näher erläutert.
Fig. 1 zeigt einen Handgriff sowie einen Anschlusseinsatz eines Endoskopschafts gemäß einem bevorzugten Ausführungsbeispiel der Erfindung und
Fig. 2 zeigt den Handgriff und den Anschlusseinsatz gemäß Fig. 1 in gekoppeltem Zustand mit einem Touchscreen als Bedienfeld.

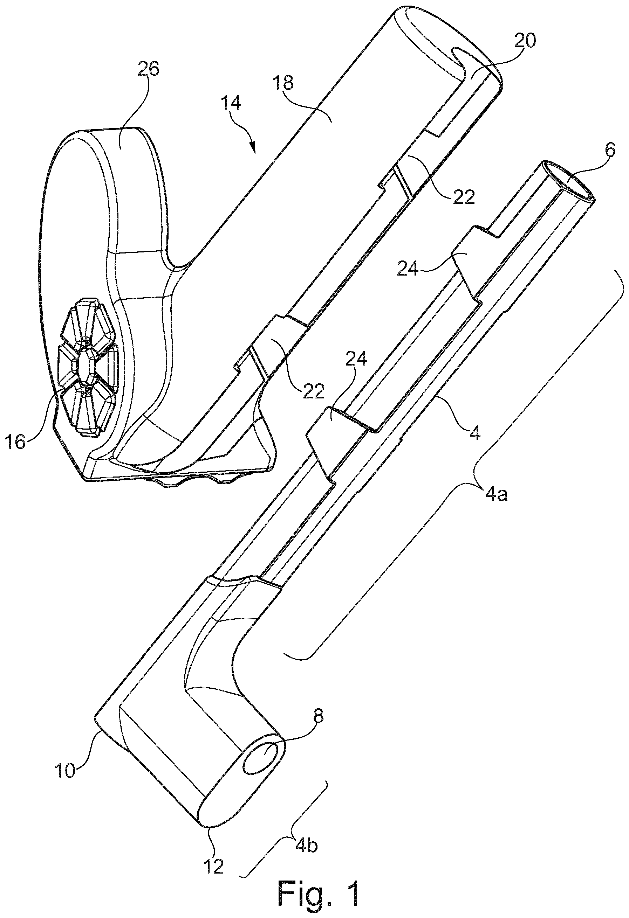

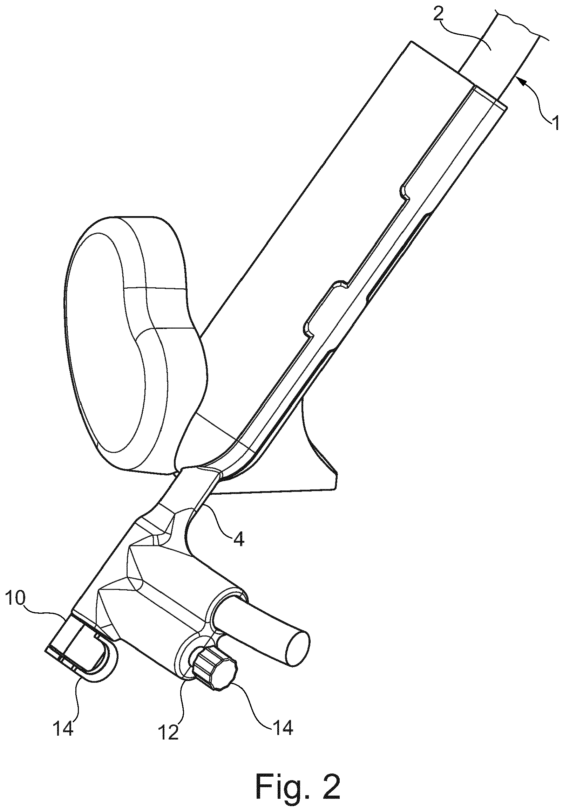

Gemäß der beiden Fig. 1 und 2 hat das Endoskop einen Endoskopschaft 1, an dessen nicht weiter dargestellten distalen Endbereich (Endoskopkopf) eine Anzahl von Funktionen verbaut ist. Diese Funktionen betreffen u. a. eine Optik, eine Beleuchtung, ggf. einen aktiv per Stellantrieb betätigbaren Abwinklungsabschnitt unmittelbar vor dem Endoskopkopf, und dergleichen. Insoweit entspricht der Endoskopschaft der vorliegenden Offenbarung dem bekannten Stand der Technik und braucht daher nicht näher beschrieben zu werden.

Im Unterschied zum allgemein bekannten Stand der Technik besteht der Endoskopschaft 1 der vorliegenden Offenbarung aus einem biegeflexiblen Schaftabschnitt 2, der dafür geeignet und dazu angepasst ist, in den Hohlraum (z.B. Darm oder Speiseröhre, etc.) eines Patienten unter passiver Nachverfolgung von Hohlraumkrümmungen/-windungen eingeführt zu werden und einem proximal daran anschließenden Anschlusseinsatz 4, der fest (oder auch lösbar) mit dem biegeflexiblen Schaftabschnitt 2 (koaxial) in axialer Verlängerung zu diesem verbunden ist. Der Anschlusseinsatz 4 besteht aus einem Material, das steifer ist als der biegeflexible Schaftabschnitt 2.

Innerhalb des Endoskopschafts 1 umfassend den flexiblen Schaftabschnitt 2 sowie den Anschlusseinsatz 4 ist ein (zentraler) Arbeitskanal 6 sowie eine Anzahl von Versorgungs-/Funktionskanälen (nicht weiter dargestellt) ausgebildet. In den Versorgungs-/Funktionskanälen sind zum Teil (elektrisch oder lichtleitende) Leitungskabel verlegt, die an die vorstehend genannten Funktionen angeschlossen sind. Es können aber auch Hydraulikflüssigkeit-führende Versorgungs-/Funktionskanäle vorgesehen sein, etwa zur hydraulischen Betätigung des Abwinklungsabschnitts oder zur Kühlung/Reinigung einzelner Funktionen im Endoskopkopf.

Diese Versorgungs-/Funktionskanäle erstrecken sich ausgehend vom Endoskopkopf durch den biegeflexiblen Schaftabschnitt 2 und den sich proximal daran anschließenden Anschlusseinsatz 4 und enden in einer Anschlusseinrichtung 8 bzw. Funktionsanschlüssen (z.B. kombinierte Elektrik/Hydraulik-Steckdose) am proximalen Ende des Anschlusseinsatzes 4.

Der Arbeitskanal 6 erstreckt sich ebenfalls ausgehend vom Endoskopkopf durch den biegeflexiblen Schaftabschnitt 2 und den Anschlusseinsatz 4 und hat einen Durchmesser, der das gleitende Hindurchführen von minimalinvasiven chirurgischen Instrumenten mit standardisierten Außendurchmessern (aus dem allgemeinen Stand der Technik bekannt) erlaubt. Der Arbeitskanal 6 öffnet sich am Endoskopkopf in Richtung distal zur Umgebung, sodass ein chirurgisches Instrument (Zange, Schere, Pinzette, etc.) aus dem Endoskopschaft vorausgeschoben werden kann.

Am proximalen Ende des Arbeitskanals mündet dieser in wenigstens zwei separate (Arbeitskanal-) Anschlüsse 10, 12, welche im Anschlusseinsatz 4 ausgebildet sind.

Im Konkreten hat der Anschlusseinsatz 4 neben den vorstehend beschriebenen Funktionsanschlüssen 8 wenigstens zwei weitere Arbeitskanalanschlüsse 10, 12, die separat sowie unabhängig voneinander (auch gleichzeitig) benutzt werden können.

Vorzugsweise ist zumindest einer der Anzahl von Arbeitskanalanschlüssen 10 dazu vorgesehen (angepasst), dass über diesen ein chirurgisches Instrument in den Arbeitskanal 6 eingeführt wird. Demzufolge hat dieser Arbeitskanalanschluss 10 wenigstens einen Durchmesser, der für das Einführen des chirurgischen Instruments (mit Standardaußendurchmesser) ausreicht. Vorzugsweise entspricht der Durchmesser dieses Arbeitskanalanschlusses dem des Arbeitskanals oder ist (etwas) größer. Des Weiteren ist dieser Arbeitskanalanschluss 10 bezüglich des Arbeitskanals 6 vorzugsweise so ausgerichtet, dass das chirurgische Instrument bei dessen Einführen in den Arbeitskanal nicht abknickt (in einem stumpfen Winkel oder axial zum Arbeitskanal 6).

Weiter vorzugsweise ist zumindest einer der Anzahl von Arbeitskanalanschlüssen 12 dazu vorgesehen (angepasst), dass über diesen ein Fluid in den Arbeitskanal 6 eingeführt oder abgeführt wird. Beispielsweise hat dieser Anschluss 12 eine Vorrichtung, die ein fluiddichtes Anschließen einer (nicht gezeigten) Pumpe, Spritze oder dergleichen ermöglicht. Eine solche Vorrichtung kann ein einfacher Rohrstutzen sein, auf den z.B. ein Schlauch aufgeschoben ist/wird oder ein Bajonett-, Schraub- oder Luerverschluss und dergleichen.

Jeder der Arbeitskanalanschlüsse 10, 12 kann ferner mit einem Verschluss (Deckel, Kappe, Blindstopfen, etc.) 14 versehen sein, mittels dem bei Nichtnutzung des jeweiligen Arbeitskanalanschlusses 10, 12 dieser fluiddicht verschlossen werden kann.

Vorzugsweise sind der eine Arbeitskanalanschluss 12 für das Ein-/Abführen von Fluid 12 und der Funktionsanschluss 8 in einem (stumpfen) Winkel (maximal 90°) zum Arbeitskanal 6 innerhalb des Anschlusseinsatzes 4 ausgerichtet. Weiter vorzugsweise verlaufen der eine Arbeitskanalanschluss 12 für das Ein-/Abführen von Fluid 12 und der Funktionsanschluss 8 parallel zueinander (auf einer Winkelposition bezüglich des Arbeitskanals 6). Sie können aber auch in Umfangsrichtung des Arbeitskanals versetzt zueinander angeordnet sein.

Insoweit bildet der Anschlusseinsatz 4 in seinem äußeren Erscheinungsbild quasi einen (distalen) Rohrlauf (in Verlängerung zum biegeflexiblen Schaftabschnitt) 4a und einen (proximalen) Anschlussstutzen 4b bestehend aus den Arbeitskanalanschlüssen 10, 12 und den weiteren (Funktions-) Anschlüssen 8 für die eingangs genannten Endoskopfunktionen.

Der so aufgebaute Endoskopschaft 1 (bestehend aus dem Anschlusseinsatz 4, dem biegeflexible Schaftabschnitt 2, ggf. dem aktiv abwinkelbaren Abschnitt und dem daran sich anschließenden Endoskopkopf - in dieser Reihenfolge) beinhaltet alle Funktionen des Endoskops sowie deren Anschlüsse 8, 10, 12 für das Anschließen an eine (nicht dargestellte) separate Betriebseinheit/-station. Eine solche Station umfasst u.a. Einrichtungen zum Betreiben der Optik und der Beleuchtung (ggf. einschließlich deren Kühlung), Einrichtungen für das Betätigen des Abwinklungsabschnitts, Einrichtungen (Pumpen) für das Einpressen von Spüllösungen oder Medikamenten und/oder das Absaugen von Flüssigkeiten, etc.

Aus diesem Grund ist es bevorzugt, den so definierten Endoskopschaft 1 als Ein-Weg-Artikel zu konzipieren, um aufwändige Reinigungsarbeiten zu vermeiden. Hier hat sich gezeigt, dass sowohl die Funktionen wie auch deren Anschlüsse preisgünstig hergestellt werden können, sodass eine Ein-Weg-Bauweise nicht nur aus hygienischen Gründen vorteilhaft ist sondern sich auch als wirtschaftlich erweist.

Das erfindungsgemäße Endoskop hat einen Handgriff 14, mittels dem das Endoskop/der Endoskopschaft 1 vorzugsweise gemäß vorstehender Definition in einer Endoskopie-Anwendung gehalten werden kann. Vorzugsweise sind im/am Handgriff 14 Betätigungselemente angeordnet, mittels denen die Funktionen des Endoskops aktuiert werden können.

Beispielsweise hat der Handgriff 14 ein Bedienfeld/Bedienerfeld 16 vorzugsweise in Form einer Tastatur, Joystick, Platte oder eines Touchscreen, die sich in ergonomisch günstiger Weise an einen Griffabschnitt 18 anschließt, derart, dass das Bedienfeld 16 mit den Fingern der zur Greifhand zweiten Hand betätigt werden kann. Optional kann alternativ oder zusätzlich zu dem Bedienfeld 16 ein (nicht weiter gezeigter) Lage-/G-Sensor/ Beschleunigungssensor bekannter Konstruktion integriert werden, um über z. B. eine Bewegung des Handgriffs die Endoskopspitze zu steuern.

Vorzugsweise ist der Griffabschnitt 18 als Halb- oder Drei-Viertelschale ausgebildet und definiert eine Art Klammer oder Manschette, die auf den Endoskopschaft 1 und vorzugsweise auf den Rohrlauf 4a des Anschlusseinsatzes 4 aufgesteckt werden kann. Hierfür hat der Griffabschnitt 18 beispielsweise eine Rohrform mit durchgehenden Längsschlitz 20, wobei im Längsschlitz 20 eine Anzahl von Zacken oder dergleichen Hinterschneidungen (Ausnehmungen) 22 ausgebildet sind. Die Rohrform ist gemäß diesem Ausführungsbeispiel im Querschnitt oval ausgebildet, um gut gehalten werden zu können und dem Greifer auch ein taktiles Gefühl für die Rotationslage des Endoskopschafts zu vermitteln. Es ist aber auch denkbar, den Griffabschnitt rund oder eckig auszubilden.

Der Anschlusseinsatz 4 hat im Bereich seines Rohrlaufs 4a ebenfalls eine Anzahl von Hinterschneidungen (Vorsprüngen) 24, die bei Einsetzen in den schalenförmigen Griffabschnitt 18 des Handgriffs 14 mit den dort ausgebildeten Hinterschneidungen 22 in Eingriff kommen und so die Relativposition des Handgriffs 14 zum Anschlusseinsatz 4 (und damit zum gesamten Endoskopschaft) festlegen.

Der Handgriff 14 bildet zumindest im Bereich des Bedienfeldes 16 ein Gehäuse/Aufnahmegehäuse 26, in dem elektrische/elektronische Bauteile zur Betätigung der separaten Betriebseinheit/-station untergebracht sind. Vorzugsweise betreffen diese elektrischen/elektronischen Bauteile eine kabellose Kommunikationseinrichtung sowie deren Energieversorgung. Als kabellose Kommunikationseinrichtung kommen beispielsweise W-Lan, Blue-Tooth oder Infrarot in Frage. Es ist aber auch denkbar, am Handgriff 14 eine Verkabelung anzuordnen, über die der Handgriff 14 (elektrisch) mit der Betriebseinheit/-station verbunden werden kann/ist. Die Energieversorgung im Handgriff 14 kann idealerweise durch eine drahtlose Energieübertragung, insbesondere durch induktive Kopplung, geladen werden. In dieser Ausführungsform kann der Handgriff 14 ohne physische elektrische Anschlüsse, mit einem komplett geschlossenen Aufnahmegehäuse 26, gestaltet werden. Der Handgriff 14 kann weiter eindeutig einer Betriebsstation zugeordnet werden, beispielsweise über eine einmalige ID, um bei einer Mehrzahl an Endoskopen in einer Umgebung nur die zugehörige Betriebsstation und damit das angeschlossene Endoskop zu aktuieren. Vorzugsweise kann der Handgriff 14 mit einer Nahfeldkommunikation als Teil der Kommunikationseinrichtung ausgestattet sein, um an einer Betriebsstation, welche ebenfalls ein elektronisches Bauteil für eine Nahfeldkommunikation aufweist, angemeldet und zugeordnet zu werden.

Das Gehäuse/Aufnahmegehäuse 26 ist dabei fluiddicht oder aber mit einer fluiddichten Hülle (vorzugsweise nur im Bereich des Bedienfeldes 16) ummantelt, sodass dessen Oberfläche, beispielsweise durch einfaches Eintauchen in eine Reinigungsflüssigkeit, gesäubert werden kann.

Optional ist am Handgriff 14 im Bereich des Griffabschnitts 18 ein Sensor oder Schalter (nicht gezeigt) vorgesehen, der den Anschlusseinsatz 4 dann registriert, wenn dieser in den Handgriff 14 (dessen Griffabschnitt 18 mit Schalenform) eingesetzt ist. Der Schalter/Sensor ist mit der Elektronik im Handgriff 14 verbunden und schaltet diese nur dann frei, wenn der Handgriff 14 korrekt mit dem Endoskopschaft 1 mechanisch gekoppelt ist. So wird das Betätigen der Endoskopfunktionen bei nicht/falsch gekoppeltem Handgriff 14 verhindert. Weiter optional ist der Handgriff für Rechts- oder Linkshänder und für unterschiedliche Handgrößen konfigurierbar.

Gemäß der anliegenden Figuren ist das Bedienfeld 16 in Form einer Scheibe/Platte ausgebildet, die sich unter einem spitzen Winkel sowie seitlich zum Griffabschnitt 18 erstreckt. Auf diese Weise kann der Griffabschnitt 18 mit einer Hand (z.B. der rechten Hand) ergriffen und parallel dazu das Bedienfeld 16 mit der anderen Hand betätigt werden.

Schließlich ist aus den Figuren zu ersehen, dass bei in den Handgriff 14 (dessen Griffabschnitt 18) eingesetztem Anschlusseinsatz 4 die daran ausgebildeten Anschlüsse (Funktionsanschlüsse und Arbeitskanalanschlüsse/-zugänge) 8-12 aus dem Handgriff 14 (dessen Griffabschnitt 18) vorzugsweise proximal herausragen. Sie sind daher auch bei eingesetztem Zustand des Anschlusseinsatzes 4 von außen frei zugänglich. Es ist somit möglich, Anschlüsse nach Einsetzen des Anschlusseinsatzes 4 in den Handgriff 14 beliebig zu nutzen oder wieder zu verschließen.

## Patentansprüche

1. Endoskop mit einem Endoskopschaft (1), an dessen distalem Ende zumindest eine Optik sowie eine Beleuchtung vorgesehen sind, die über Funktions- und/oder Versorgungskanäle innerhalb des Endoskopschafts (1) an eine Betriebsstation anschließbar sind, und
mit einem am distalen Ende des Endoskopschafts (1) sich öffnenden Arbeitskanal (6), der im Endoskopschaft (1) ausgeformt und dafür angepasst ist, chirurgische Instrumente der Minimalinvasivbauart längsverschieblich aufzunehmen, und
mit einem Handgriff (14) mit einem Griffabschnitt (18) und einer Kommunikationseinrichtung zur kabellosen Kommunikation mit der Betriebsstation,
wobei an dem Handgriff (14) ein Aufnahmegehäuse (26) angeformt ist, in welchem elektrische und/oder elektronische Bauteile der Kommunikationseinrichtung untergebracht sind, und an dessen Außenseite ein Bedienfeld (16), vorzugsweise in Form einer Tastatur oder eines Touchscreens, angeordnet ist, das mit der Kommunikationseinrichtung in elektrischer Verbindung steht,
wobei der Handgriff (14) als ein zum Endoskopschaft (1) separates Bauteil vorgesehen ist,
wobei der Endoskopschaft (1) zumindest einen biegeflexiblen Abschnitt (2) und einen proximalen vergleichsweise starren Anschlusseinsatz (4) hat, an dem Anschlüsse (8 bis 12) für die Funktions- und/oder Versorgungskanäle sowie des Arbeitskanals (6) ausgebildet oder angeordnet sind,
wobei der Anschlusseinsatz (4) einen Rohrlauf (4a) hat, dessen eines Ende mit dem Arbeitskanal (6) innerhalb des mit dem Anschlusseinsatz (4) fest verbundenen biegeflexiblen Schaftabschnitts (2) fluidverbunden ist, und an dessen anderem Ende ein Anschlussstutzen (4b) angeordnet ist, an dem die Anschlüsse (8 bis 12) für die Funktions- und/oder Versorgungskanäle sowie des Arbeitskanals (6) vorgesehen sind,
**dadurch gekennzeichnet, dass** der Griffabschnitt (18) eine Rohrform mit durchgehendem Längsschlitz (20) hat, wobei im Längsschlitz (20) eine Anzahl von Hinterschneidungen oder Ausnehmungen (22) ausgebildet ist, und
der Anschlusseinsatz (4) im Bereich des Rohrlaufs (4a) eine Anzahl von Hinterschneidungen oder Vorsprüngen (24) hat, die bei Einsetzen in den Griffabschnitt (18) mit den dort ausgebildeten Hinterschneidungen (22) in Eingriff kommen und so die Relativposition des Handgriffs zum Anschlusseinsatz festlegen.

2. Endoskop nach Anspruch 1, **dadurch gekennzeichnet, dass** an dem Griffabschnitt (18) das Aufnahmegehäuse (26) stoffeinstückig angeformt ist.

3. Endoskop nach einem der Ansprüche 1 oder 2, **dadurch gekennzeichnet, dass** im Aufnahmegehäuse (26) eine Energiequelle, vorzugsweise in Form einer Batterie, zur Stromversorgung der Kommunikationseinrichtung und vorzugsweise des Bedienfelds (16) untergebracht ist.

4. Endoskop nach einem der Ansprüche 1 bis 3, **dadurch gekennzeichnet, dass** das Aufnahmegehäuse (26) und das Bedienfeld (16) nach außen fluiddicht verschlossen sind, sodass der Handgriff (14) für eine Nassreinigung, vorzugsweise durch ein Tauchverfahren, angepasst ist.

5. Endoskop nach einem der vorstehenden Ansprüche, **dadurch gekennzeichnet, dass** durch die durchgehend längsgeschlitzte Rohrform des Griffabschnitts (18) eine Clipfunktion verwirklicht wird, die ein Aufklippen des Handgriffs (14) auf den Endoskopschaft (1) des Endoskops für dessen mechanische Verbindung mit dem Endoskopschaft (1) ermöglicht.

6. Endoskop nach einem der vorstehenden Ansprüche, **dadurch gekennzeichnet, dass** der Handgriff (14) zumindest im Bereich des Aufnahmegehäuses (26) und vorzugsweise des Bedienfeldes (16) mit einer fluiddichten Hülle ummantelt ist, wobei optional zusätzlich zu dem Bedienfeld (16) ein Lagesensor zur handgriffsbewegungsabhängigen Steuerung des Endoskops innerhalb des Handgriffs (14) vorgesehen ist.

7. Endoskop nach einem der vorstehenden Ansprüche, **dadurch gekennzeichnet, dass** der Griffabschnitt (18) federelastisch radial aufspreizbar ist.

8. Endoskop nach einem der vorstehenden Ansprüche, **dadurch gekennzeichnet, dass** bei in den Handgriff (14) eingesetztem Rohrlauf (4a) die Anschlüsse (8, 10, 12) des Anschlussstutzens (4b) zumindest abschnittsweise aus dem Handgriff (14) herausragen oder von außen zugänglich sind.

## Claims

1. Endoscope with an endoscope shaft (1), at the distal end of which at least one optics as well as an illumination are provided, which can be connected to an operating station via functional and/or supply channels within the endoscope shaft (1), and
with a working channel (6) which opens at the distal end of the endoscope shaft (1), and which is formed in the endoscope shaft (1) and adapted to receive surgical instruments of the minimally invasive type in a longitudinally displaceable manner, and
with a handle (14) with a handle portion (18) and a communication device for wireless communication with the operating station,
wherein an accommodation housing (26), in which electrical and/or electronic components of the communication device are accommodated, is formed on the handle (14), and on the outside of which a control panel (16), preferably in the form of a keyboard or a touch screen, is arranged, the control panel being electrically connected to the communication device,
wherein the handle (14) is provided as a component separate from the endoscope shaft (1),
wherein the endoscope shaft (1) has at least one flexurally flexible section (2) and a proximal, comparatively rigid connection insert (4) on which connections (8 to 12) for the functional and/or supply channels and the working channel (6) are formed or arranged,
wherein the connection insert (4) has a pipe rail (4a), one end of which is fluidly connected to the working channel (6) within the flexurally flexible shaft portion (2) fixedly connected to the connection insert (4), and at the other end of which a connection nozzle (4b) is arranged on which the connections (8 to 12) for the functional and/or supply channels and the working channel (6) are provided,
**characterized in that** the handle portion (18) has a pipe shape with a continuous longitudinal slot (20), wherein a number of undercuts or recesses (22) is formed in the longitudinal slot (20), and the connection insert (4) has in the area of the pipe rail (4a) a number of undercuts or projections (24) which, when inserted into the handle portion (18), engage with the undercuts (22) formed there and thus determine the relative position of the handle to the connection insert.

2. Endoscope according to claim 1, **characterized in that** the accommodation housing (26) is integrally formed on the handle portion (18).

3. Endoscope according to one of claims 1 or 2, **characterized in that** an energy source, preferably in the form of a battery, is accommodated in the accommodation housing (26) for powering the communication device and preferably the control panel (16).

4. Endoscope according to one of claims 1 to 3, **characterized in that** the accommodation housing (26) and the control panel (16) are closed fluid-tight to the outside so that the handle (14) is adapted for wet cleaning, preferably by a dipping method.

5. Endoscope according to one of the preceding claims, **characterized in that** a clip function is realized by the continuously longitudinally-slotted pipe shape of the handle portion, which allows the handle (14) to be clipped onto the endoscope shaft (1) of the endoscope for its mechanical connection to the endoscope shaft (1).

6. Endoscope according to one of the preceding claims, **characterized in that** the handle (14) is surrounded by a fluid-tight sleeve at least in the region of the accommodation housing (26) and preferably of the control panel (16), wherein optionally, in addition to the control panel (16), a position sensor is provided for controlling the endoscope within the handle (14) as a function of handle movement.

7. Endoscope according to one of the preceding claims, **characterized in that** the handle portion (18) can be spring-elastically spread radially.

8. Endoscope according to one of the preceding claims, **characterized in that,** when the pipe rail (4a) is inserted in the handle (14), the connections (8, 10, 12) of the connection nozzle (4b) protrude at least in sections from the handle (14) or are accessible from the outside.

## Revendications

1. Endoscope avec une tige d'endoscope (1), à l'extrémité distale de la laquelle sont prévus une optique ainsi qu'un éclairage, qui peuvent être raccordés par l'intermédiaire de canaux de fonctionnement et/ou d'alimentation à l'intérieur de la tige d'endoscope (1) à un poste de commande et
avec un canal de travail (6) s'ouvrant au niveau de l'extrémité distale de la tige d'endoscope (1), qui est formé dans la tige d'endoscope (1) et conçu pour loger, de manière coulissante longitudinalement, des instruments chirurgicaux du type minimalement invasif, et
avec une poignée (14) avec une portion de poignée (18) et un dispositif de communication pour une communication sans fil avec le poste de commande,
un boîtier de logement (26) étant formé sur la poignée (14), dans lequel des composants électriques et/ou électroniques du dispositif de communication sont logés, et sur le côté extérieur duquel un panneau de commande (16), de préférence sous la forme d'un clavier ou d'un écran tactile, est disposé, qui est relié électriquement avec le dispositif de communication,
la poignée (14) étant conçue comme un composant séparé de la tige d'endoscope (1),
la tige d'endoscope (1) comprenant au moins une portion flexible (2) et un insert de raccordement (4) relativement proximal, sur lequel des raccordements (8 à 12) pour les canaux de fonctionnement et/ou d'alimentation ainsi que du canal de travail (6) sont réalisés ou disposés,
l'insert de raccordement (4) comprenant un tube (4a), dont une extrémité est reliée de manière fluidique avec le canal de travail (6) à l'intérieur de la portion de tige flexible (2) reliée fermement avec l'insert de raccordement (4), et à l'autre extrémité duquel est disposé un manchon de raccordement (4b) sur lequel sont prévus les raccordements (8 à 12) pour les canaux de fonctionnement et/ou d'alimentation ainsi que du canal de travail (6),
**caractérisé en ce que** la portion de poignée (18) présente une forme tubulaire avec une fente longitudinale continue (20), moyennant quoi, dans la fente longitudinale (20) sont réalisés plusieurs contre-dépouilles ou évidements (22), et l'insert de raccordement (4) comprend, au niveau du tube (4a), plusieurs contre-dépouilles ou saillies (24) qui s'emboîtent, lors de l'insertion dans la portion de poignée (18) avec les contre-dépouilles (22) qui y sont réalisées et fixent ainsi la position relative de la poignée par rapport à l'insert de raccordement.

2. Endoscope selon la revendication 1, **caractérisé en ce que** le boîtier de logement (26) est moulé d'une seule pièce sur la portion de poignée (18).

3. Endoscope selon l'une des revendications 1 ou 2, **caractérisé en ce que**, dans le boîtier de logement (26), est logée une source d'énergie, de préférence sous la forme d'une batterie, pour l'alimentation électrique du dispositif de communication et de préférence du panneau de commande (16).

4. Endoscope selon l'une des revendications 1 à 3, **caractérisé en ce que** le boîtier de logement (26) et le panneau de commande (16) sont scellés de manière étanche aux fluides vers l'extérieur de façon à ce que la poignée (14) soit adaptée à un nettoyage humide, de préférence par un procédé par immersion.

5. Endoscope selon l'une des revendications précédentes, **caractérisé en ce que** la forme tubulaire à fente longitudinale continue de la portion de poignée (18) permet de réaliser une fonction de clipsage, qui permet un clipsage de la poignée (14) sur la tige d'endoscope (1) de l'endoscope pour sa liaison mécanique avec la tige d'endoscope (1).

6. Endoscope selon l'une des revendications précédentes, **caractérisé en ce que** la poignée (14) est entourée, au moins au niveau du boîtier de logement (26) et de préférence du panneau de commande (16), par une enveloppe étanche aux fluides, moyennant quoi, en option, en plus du panneau de commande (16), un capteur de position est prévu pour le contrôle de l'endoscope à l'intérieur de la poignée (14) en fonction des mouvements de la poignée.

7. Endoscope selon l'une des revendications précédentes, **caractérisé en ce que** la portion de poignée (18) peut être écartée radialement de manière élastique.

8. Endoscope selon l'une des revendications précédentes, **caractérisé en ce que**, lorsque le tube (4a) est inséré dans la poignée (14), les raccordements (8, 10, 12) du manchon de raccordement (4b) dépassent au moins à certains endroits de la poignée (14) ou sont accessibles de l'extérieur.
